# EUROPÄISCHE PATENTSCHRIFT

(11) **EP 2 852 335 B1**
(45) Veröffentlichungstag und Bekanntmachung des Hinweises auf die Patenterteilung: **23.12.2015**
(21) Anmeldenummer: 14739383.9
(22) Anmeldetag: 08.07.2014
(51) Int. Cl.: A61B 17/122, A61B 17/00

(54) **CHIRURGISCHER CLIP, INSBESONDERE ANEURYSMENCLIP**
SURGICAL CLIP, IN PARTICULAR ANEURYSM CLIP
CLIP CHIRURGICAL, EN PARTICULIERS CLIP D'ANÉVRISME

(30) Priorität: 23.07.2013 DE 102013107876
(43) Veröffentlichungstag der Anmeldung: 01.04.2015
(73) Patentinhaber: Aesculap AG, 78532 Tuttlingen (DE)
(72) Erfinder: ZIERIS, Gerold, 78570 Mühlheim (DE); MOTZ, Corvin, 88630 Pfullendorf (DE); AMANN, Joachim, 78357 Mühlingen - Zoznegg (DE)
(74) Vertreter: Winter, Brandl, Fürniss, Hübner, Röss, Kaiser, Polte - Partnerschaft mbB
(86) Internationale Anmeldenummer: PCT/EP2014/064610
(87) Internationale Veröffentlichungsnummer: WO 2015/010893

(56) Entgegenhaltungen:
- WO-A2-2004/080275
- DE-A1- 10 309 491
- GB-A- 2 161 206

## Beschreibung

Die vorliegende Erfindung betrifft einen Chirurgischen Clip, insbesondere einen Aneurysmenclip, mit einem ersten Klemmarm bzw. Maulteil und einem zweiten Klemmarm bzw. Maulteil, die mittels eines Scharniers drehbar miteinander gekoppelt sind und mittels einer Feder in der Drehrichtung gegeneinander in eine Schließstellung vorgespannt sind.

### Hintergrund der Erfindung

Chirurgische Clips sind medizinische Instrumente oder Implantate, die temporär zum Klemmen oder Verschließen von Gewebeperforationen oder zur langzeitlichen Therapie beispielsweise von Aneurysmen als Gefäßklammern eingesetzt werden. Es ist eine Vielzahl unterschiedlicher Clips bekannt, zum Beispiel in Form von maulartigen Clips, bei denen Klemm- oder Clipbranchen ähnlich wie Ober- und Unterkiefer einander gegenüberliegend und in Längsrichtung gebogen ausgebildet und jeweils an ihren beiden Enden über ein Scharnier miteinander gekoppelt sind. Ein anders Beispiel sind schmale, langgestreckte Clips, vergleichbar zu einer herkömmlichen Zange, mit zwei gegenüberliegenden Klemmarmen (Branchen), die ggf. mit Zähnen oder einer Riffelung versehen sind und an jeweils einem Ende miteinander scharnierartig gekoppelt sind.

### Stand der Technik

Clips nach dem Stand der Technik haben in der Regel zwei scharnierartig miteinander gekoppelte Maulteile, die jeweils einen Klemmabschnitt (nachfolgend auch als Klemmarm oder Branche bezeichnet) und einen Betätigungsabschnitt ausbilden. Ferner weisen die Clips allgemein eine Federanordnung auf, die einer Vorspannung des Clips in seine geschlossene Stellung dient und eine durch die Clipbranchen oder Klemmarme ausgeübte Klemmkraft bewirkt. Es sind sowohl Clips bekannt, bei denen die Federanordnung ein separates, mit den Maulteilen des Clip zusammenwirkendes Bauteil ist, als auch Clips, bei denen die Federanordnung integraler Bestandteil des Clips bzw. der Maulteile ist.

Es sind Aneurysmenclips bekannt, bei denen die Branchen oder Klemmarme mittels einer U-förmigen Schenkelfeder gegeneinander vorgespannt sind. Deren Schenkel greifen entweder direkt von außen oder seitlich an den Klemmarmen an und pressen diese zusammen, oder sie greifen an, die Klemmarme in Cliplängsrichtung jeweils verlängernden Betätigungsabschnitten an und pressen diese zusammen oder auseinander, abhängig davon, ob die Maulteile sich im Scharnierbereich überkreuzen oder parallel sind.

Bei Verwendung einer Schenkelfeder ergeben sich jedoch Nachteile. Schenkelfedern insbesondere mit spiralförmig gewickeltem Energiespeicher erfordern in der Regel eine vergleichsweise aufwändige und komplexe Herstellung. Insbesondere beim Wickeln der Feder darf keine Rissbildung im Federmaterial erfolgen. Ebenso ist ein Überwickeln der Schenkelfeder zu vermeiden, da sich ansonsten die Anpresskraft zwischen den Klemmarmen verringern kann. Insgesamt erfordert die Herstellung der Schenkelfeder ein hohes Maß an Präzision, um Instrumente herzustellen, deren Eigenschaften wie Anpresskraft, Haltbarkeit, Betriebssicherheit, etc. innerhalb eines engen Toleranzbereichs liegen.

Ein weiteres Problem bei Verwendung von Schenkelfedern ist das Vorsehen einer reproduzierbaren Vorspannkraft. Die Schenkelfeder muss so gestaltet und dimensioniert sein, dass eine vorbestimmte Vorspannkraft auf die Klemmabschnitte ausgeübt wird. Diese ist ausschlaggebend für die maximal erreichbare Schließkraft. Bei vorgegebenen Abmessungen des Clips kann unter Umständen das Erreichen der gewünschten Schließkraft problematisch sein und ist zumindest oft nur unter hohem Aufwand zu erreichen. Auch kann eine plastische Verformung des Federbereichs auftreten, insbesondere bei einer geringen Wicklungszahl oder einer reinen U-Form der Schenkelfeder, wobei die Wicklungszahl je nach zur Verfügung stehendem Bauraum zwischen 0,5 und 2 liegt.

Da eine Schenkelfeder relativ zu den Branchen des Clips bzw. zu dessen Klemmarmen in der Regel asymmetrisch angeordnet ist, können mit Nachteil Reibungskräfte zwischen den Branchen bzw. Klemmarmen und den damit zusammenwirkenden Schenkeln der Feder auftreten. Dies führt zu einer Verringerung der maximal erreichbaren Schließkraft. Die asymmetrische Anordnung kann außerdem bewirken, dass die Klemmarme des Clips nicht parallel öffnen bzw. schließen. Ein weiterer Nachteil ist die in der Regel nur linienförmige Anlage der Schenkel der Feder an den Branchen.

Aus der DE 10 2011 055 094 A1 ist ein Aneurysmenclip bekannt, der ein erstes Maulteil mit einem Klemmarm aufweist, an dessen einem proximalen Endbereich ein Gabelabschnitt ausgeformt ist. Der Clip weist des Weiteren ein zweites Maulteil mit einem Klemmarm auf, an dessen proximalen Ende eine Spiralfeder ausgebildet ist. Diese ist in den Gabelabschnitt des ersten Maulteils so eingesetzt und darin geführt, dass sie mit dem Gabelabschnitt drehfest gekoppelt ist. Diese Verwendung einer Spiralfeder vermeidet einige der vorgenannten Nachteile von Schenkelfedern. Allerdings kommt bei diesem Clip wie auch bei den vorstehend beschriebenen Clips die Schenkelfeder mit Körpergewebe in Kontakt, so dass der Werkstoff der Feder hinsichtlich Körperverträglichkeit und Hygiene die gleichen Anforderungen erfüllen muss wie der Werkstoff der Maulteile.

Die Druckschrift GB 2 161 206 offenbart einen chirurgischen Clip mit den Merkmalen des Oberbegriffs von Anspruch 1.

### Kurzbeschreibung der Erfindung

Ausgehend von dem vorstehend beschriebenen Stand der Technik liegt der Erfindung die Aufgabe zugrunde, einen chirurgischen Clip, insbesondere einen Aneurysmenclip insbesondere von schlanker Gestalt zu schaffen, der einfach und mit möglichst geringen Einschränkungen applizierbar und gut handhabbar sowie zu reinigen ist. Ein Ziel ist es, einen chirurgischen Clip (Aneurysmenclip) bereit zu stellen, bei dem ein Erreichen einer definierten Vorspannkraft gewährleistet ist, Ferner wäre es vorteilhaft, wenn plastische Verformungen der Feder oder Teilbereiche der Feder vermieden werden können und ein definiertes Öffnen und Schließen der Klemmarme ohne Verkanten und Asymmetrie möglich ist.

Diese Aufgabe sowie die weitere Zielsetzung wird gelöst durch einen chirurgischen Clip oder Gewebeclip, insbesondere einen Aneurysmenclip mit den Merkmalen gemäß dem Patentanspruch 1.

Insbesondere wird ein chirurgischer Clip (Aneurysmenclip) bereit gestellt, mit
einem ersten und einem zweiten Maulteil, die drehbar miteinander gekoppelt sind, insbesondere mittels eines Scharniers, und mittels einer Feder in der Drehrichtung gegeneinander in eine Clip - Schließstellung vorgespannt sind, wobei an dem ersten Maulteil ein erster Gehäuseabschnitt und an dem zweiten Maulteil ein zweiter Gehäuseabschnitt ausgebildet oder angeordnet sind, welche für die Ausbildung eines nach Außen im Wesentlichen geschlossenen Gehäuses zusammenwirken, in dem die Feder aufgenommen ist.

Jedes Maulteil des Clip hat vorzugsweise einen Klemmarmabschnitt bzw. Branche, die jeweils in einem (distalen) Abschnitt weiter vorzugsweise schalenartig zu einem Gehäuseteil ausgebildet sind. Nach einer Ausführungsform können der erste Gehäuseteil und/oder der zweite Gehäuseteil aus einer zylinderförmigen Wand mit jeweils einer geschlossenen Stirnseite (d.h. becherartig) ausgebildet sein. Die Gehäuseteile der Klemmarme bzw. der beiden Maulteile passen zu-/ineinander und bilden das Gehäuse aus, in dem die Feder innenliegend angeordnet ist. Insbesondere können die Gehäuseabschnitte und damit das gesamte Gehäuse sowie die Feder zu einer quer zur Drehachse des Clips verlaufenden Ebene symmetrisch ausgebildet sein, so dass eine Belastung der Feder nur quer zur Drehachse sowie ein symmetrisches Einleiten von Federkräften in die Branchen ohne Querkraftkomponenten in Richtung der Drehachse des Clips sichergestellt ist.

Mit Vorteil ist die Feder in dem Gehäuse gegenüber der Umgebung abgeschirmt. Das Federmaterial braucht daher nicht die gleichen Anforderungen zu erfüllen wie das Material der Maulteile oder des Gehäuses, sondern kann spezifisch auf Federeigenschaften optimiert ausgesucht und verwendet werden. Die Feder ist mit besonderem Vorteil in dem Gehäuse geschützt. Eine Beschädigung der Feder kann auf diese Weise effektiv vermieden werden. Nach außen sichtbar bzw. nach außen weisend sind nur die beiden Klemmarme des Clips, ggf. die beiden Betätigungsabschnitte, die sich in proximaler Verlängerung zu den Klemmarmen an den Gehäuseteilen anschließen, sowie die Außenseite des die Feder umhüllenden Gehäuses. Es können so besonders einfach glatte Oberflächen ohne Vertiefungen, Kanten, Hinterschneidungen oder Ähnliches ausgebildet werden, in denen sich Verunreinigungen ansammeln können. Des Weiteren ist ein Clip mit einfacher, möglichst glatter Außengestalt mit Vorteil einfach zu Applizieren.

Die Feder kann mit einem ersten Feder-Endbereich in dem Gehäuse an einer der beiden Klemmarme/Maulteile drehfest angeordnet und vorzugsweise gehalten sein. Mit dem gegenüberliegenden Feder-Endbereich ist sie an dem andern Klemmarm/Maulteil innerhalb des Gehäuses (lose) abgestützt, beispielsweise gegen einen Vorsprung abgestützt oder in einer Ausnehmung aufgenommen. Beim Zusammenfügen der beiden Maulteile (oder Maulteilhälften) wird die in/an einer der beiden Maulteile (fest-)gehaltene Feder in die zum Erreichen der gewünschten Klemmkraft erforderliche Vorspannung vorgespannt, insbesondere indem das andere Federende unter elastischer Verformung der Feder mit dem Vorsprung/Ausnehmung des anderen Maulteils in Wirkeingriff gebracht wird. Mit besonderem Vorteil wird so eine einfach Montage des Clips ohne zwingende Verwendung zusätzlicher Werkzeuge ermöglicht. Außerdem kann die erwünschte Klemmkraft des Clip durch geeignete Auswahl der Feder oder Veränderung der Vorspannung zum Beispiel durch entsprechende Platzierung von die Feder abstützenden Elementen (Vorsprung, Ausnehmung, etc) erzielt werden und ist insbesondere leicht reproduzierbar.

Nach einer Ausführungsform kann der erste Gehäuseteil an dem ersten Klemmarm endseitig ausgebildet oder angeordnet sein und/oder der zweite Gehäuseteil an dem zweiten Klemmarm endseitig ausgebildet oder angeordnet sein. Auf diese Weise wird ein schlanker, langgestreckter Clip ermöglicht, der besonders wenig Raum einnimmt und unter beengten Verhältnissen einfach handhabbar ist. Vorzugsweise sind beide Gehäuseabschnitte fluchtend mit den Klemmarmen der Branchen angeordnet.

In einer Ausführungsform kann der Clip einen Achsstift aufweisen, der im Inneren des Gehäuses ausgebildet oder angeordnet ist. Er kann fest an einem der beiden Gehäuseteile angeordnet oder einteilig mit diesem ausgebildet sein, während er in eine entsprechende Aufnahme (Loch) des anderen Gehäuseteils eingreift.

Der Achsstift kann mit Vorteil mehrere Zwecke erfüllen. Er kann zum Einem die beiden Maulteile bzw. Klemmarme zueinander zentrieren und halten. Zum Anderen kann er die Drehachse des Scharniers ausbilden. Und schließlich kann er nach einer Weiterbildung der Erfindung eine ggf. drehfeste Fixierung der Spiral- Feder bewirken. Zu diesem Zweck kann der Achsstift eine Aufnahme, vorzugsweise in Form einer Ausnehmung oder eines Schlitzes aufweisen. In diesem ist in vorteilhafter Weise ein Endabschnitt der Feder aufgenommen und gehalten. Nach einer Form der Erfindung ist der Achsstift zu einem der beiden Gehäuseabschnitte drehfest und zum anderen der beiden Gehäuseabschnitte bezogen auf die Drehachse des Scharniers drehbar.

Alternativ oder zusätzlich zu dem Achsstift kann das Scharnier des Clips durch die Gehäusewand oder durch Abschnitte der Gehäusewand selbst ausgebildet sein. Zu diesem Zweck weist nach einer Ausführungsform die zylinderförmige Wand des ersten Gehäuseteils und des zweiten Gehäuseteils jeweils Lagerflächen oder -abschnitte auf, die ineinander greifen oder zusammenwirken und das Scharnier ausbilden.

Der Clip kann die Betätigungsabschnitte oder Betätigungselemente, beispielsweise in Form von Nasen, Vorsprüngen oder dergleichen, aufweisen. Diese können unter Anderem einem Öffnen des Clips gegen die Vorspannung der Feder dienen und sind vorzugsweise an dem ersten Gehäuseteil und/oder dem zweiten Gehäuseteil angeordnet oder ausgebildet. Die Betätigungselemente können außerdem derart ausgebildet sein, dass sie einen maximalen Öffnungswinkel des Clips definieren und begrenzen. Auf diese Weise wird mit Vorteil ein Überdehnen der Feder verhindert, so dass ein vorzeitiges Ermüden der Feder weitgehend verhindert werden kann und auch nach längerer Gebrauchsdauer das Erreichen der gewünschten Schließkraft sichergestellt ist.

Erfindungsgemäß ist die Feder eine Spiralfeder. Dadurch ist ein vergleichsweise großer Federweg auch bei kleiner äußerer Abmessung der Feder garantiert, so dass der Clip weit zu öffnen ist, ohne die Feder plastisch zu überbiegen oder zu brechen. Erfindungsgemäß ist die Spiralfeder in Form einer ebenen Spirale, die sich besonders gut symmetrisch zu den Klemmarmen des Clips und insbesondere in deren Längsrichtung fluchtend anordnen lässt. Dies bewirkt, dass sich die Klemmarme im Wesentlichen parallel öffnen und schließen.

Mit besonderem Vorteil hat die Feder die Form einer Archimedischen Spirale, so dass zwischen aktiven Windungen der Feder ein konstantes Spiel vorliegt. Das Gehäuse aus dem ersten und dem zweiten Gehäuseabschnitt bildet mit Vorteil eine seitliche, vorzugsweise beidseitige Führung für die Feder aus. Diese kann bei einem Öffnen oder Schließen des Clips, wobei sich die Spiralfeder elastisch deformiert, nämlich aufweitet bzw. zusammenzieht, nicht seitlich ausbauchen oder verkanten, was einer vorzeitigen Materialermüdung der Feder entgegenwirkt.

Nach einer weiteren Form der Erfindung kann die Feder einen rechteckigen Querschnitt aufweisen, was eine definierte Richtung der durch die Feder bewirkten Vorspannkraft sicherstellt und einem parallelen Öffnen und Schließen der Klemmarme des Clips zugute kommt. Der rechteckige Federdrahtquerschnitt wirkt auch seitlichem Ausbauchen der Feder unter Biegespannung entgegen. Schließlich kann eine Spiralfeder mit rechteckigem Federdrahtquerschnitt besonders einfach in einem Arbeitsgang, beispielsweise durch Laserschneiden oder Stanzen hergestellt werden, so dass die gewünschten Federeigenschaften und die durch diese bedingten Eigenschaften des Clips wie z.B. definierte reproduzierbare Vorspannkraft einfach, verlässlich und präzise vorgesehen werden können.

Vorzugsweise hat die Spiralfeder 1.5 bis 3 Windungen, um einen ausreichenden Öffnungswinkel für die beiden Branchen bzw. Klemmarme (oder Klemmschienen) sicher zu gewährleisten. Da die Spiralfeder, wie vorstehend angedeutet wurde, durch einen maschinellen, ggf. Material abtragenden Fertigungsprozess wie Schneiden, Stanzen, etc. herstellbar ist, können kleine Fertigungstoleranzen bei geringer Ausschussrate realisiert werden, was den Clip insgesamt preisgünstig macht, die Stückzahl erhöht und die Betriebssicherheit steigert.

Insgesamt hat ein Clip nach der vorliegenden Erfindung bei großer Federkraft nur einen geringen Raumbedarf, was ihn universell und in weiten Bereichen der chirurgischen Anwendungen brauchbar macht. Im Vergleich zu einem Clip nach dem Stand der Technik ist ein deutlich geringerer Bauraum erforderlich. Des Weiteren kann die Fläche, über die die Feder an den Maulteilen/Klemmarmen abgestützt ist, bei der Erfindung wesentlich größer sein als bei Verwendung einer Schenkelfeder.

Durch die Erfindung wird ermöglicht, dass Feder und Maulteile (insbesondere Klemmarme bzw. Branchen) aus unterschiedlichen Werkstoffen bestehen können. Die Maulteile/Klemmarme können z.B. aus Kunststoff oder einem Keramikmaterial bestehen und insbesondere im Spitzgussverfahren oder mit ähnlichen Verfahren hergestellt werden. Auf diese Weise ist es möglich, auch Klemmarme mit komplexen Geometrien, z.B. Bajonett oder Fenster, einfach und kostengünstig herzustellen. Auch sind Clips möglich, die Artefakte bei einem CT- oder MRT-Verfahren vermeiden. Für die Feder können hingegen Werkstoffe optimal nur hinsichtlich der Federeigenschaften ausgewählt werden.

### Figurenbeschreibung

Weitere Merkmale und Vorteile der vorliegenden Erfindung ergeben sich aus der folgenden beispielhaften Beschreibung einer besonders bevorzugten Ausführungsform der Erfindung anhand der Figuren. Dabei zeigt:
**Fig. 1** eine Ausführungsform eines chirurgischen Clips nach der Erfindung in einer perspektivischen, teilgeschnittenen Ansicht,
**Fig. 2** eine Ausführungsform eines chirurgischen Clips nach der Erfindung in einer perspektivischen Ansicht teilweise demontiert und
**Fign. 3a,b,c** den Clip der Fig. 2 in drei perspektivischen Teilansichten in unterschiedlichen Montagestadien.

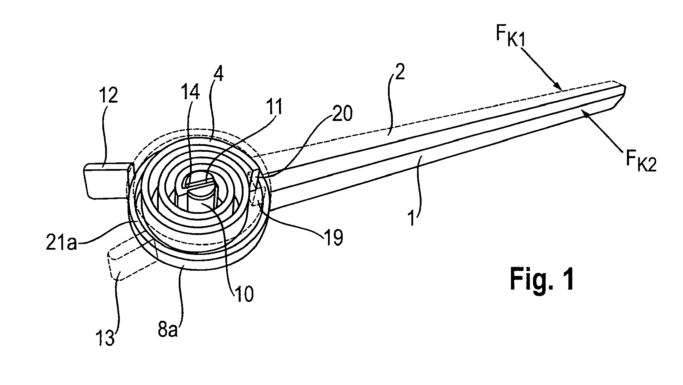

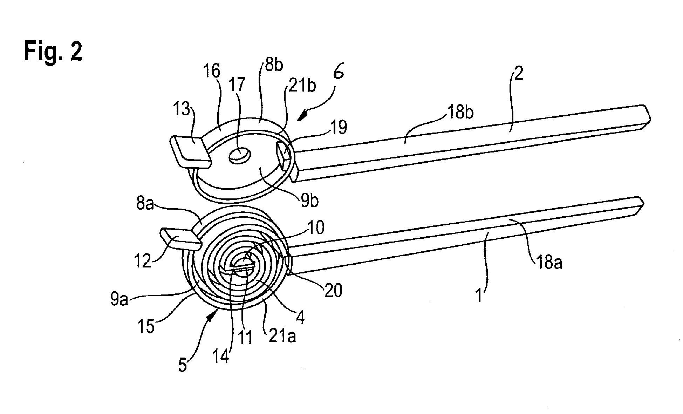

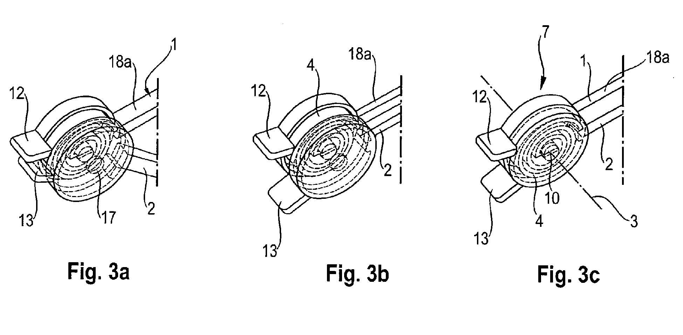

Der in den Figuren 1 und 2 gezeigte chirurgische Clip ist in einer teilgeschnittenen bzw. teildemontierten Darstellung wiedergegeben. Der Clip weist demnach zwei Maulteile (Maulteilhälften) auf, nämlich ein erstes Maulteil 15 mit einem ersten Klemmarm (oder Branche) 1 und ein zweites Maulteil 16 mit einem zweiten Klemmarm (oder Branche) 2. Die beiden Maulteile 15 und 16 und damit der Klemmarm 1 und der Klemmarm 2 sind dreh- bzw. schwenkbar miteinander gekoppelt und mittels einer Feder 4 in eine Drehrichtung gegeneinander in eine geschlossene Clip-Stellung vorgespannt. In dieser Stellung, in der die Klemmarme 1 und 2 mit einer gewünschten Klemmkraft gegeneinander gepresst sind, ist der Clip in Figur 1 sowie in Figur 3c gezeigt. Die zwischen den Klemmarmen 1 und 2 wirkende Klemmkraft ist in der Figur 1 mit Pfeilen F_{K1} und F_{K2} angedeutet. Die Klemmarme 1 und 2 sind jeweils langgestreckt, mit im zusammengefügten Zustand - dargestellt in den Figuren 1 und 3c - zueinander weisenden ebenen Klemmflächen 18a,b ausgebildet und verjüngen sich in Richtung ihres jeweiligen freien (distalen) Endes hin (in der Figur 1 in Richtung nach links). Die Klemmflächen 18a,b sind in der dargestellten Ausführungsform glatt ausgebildet, können jedoch auch geeignet profiliert, zum Beispiel mit einer Zahn- oder Rillenstruktur ausgebildet sein.

Das erste Maulteil 15 weist einen ersten Gehäuseabschnitt oder Gehäuseteil 5 auf, der an dem proximalen Ende des ersten Klemmarms 1 ausgebildet oder angeordnet ist. Das zweite Maulteil 16 weist einen zweiten Gehäuseabschnitt oder Gehäuseteil 6 auf, der an dem proximalen Ende des zweiten Klemmarms 2 ausgebildet oder angeordnet ist. In Fig. 1 ist der Gehäuseabschnitt 6 des zweiten Maulteils 16 aufgeschnitten dargestellt und nur angedeutet. In den Figuren 3a bis 3c ist der Gehäuseabschnitt 6 durchsichtig dargestellt, um die Lage und Anordnung von durch den Gehäuseabschnitt 6 verdeckten Einheiten des Clips zu verdeutlichen.

Die Gehäuseabschnitte 5 und 6 sind, wie vorstehend bereits angedeutet, jeweils endseitig, dem freien (distalen) Ende des Klemmarms 1 bzw. 2 gegenüberliegend, an dem jeweiligen Klemmarm 1 bzw. 2 ausgebildet oder angeordnet. Sie bilden bei zusammengefügten Maulteilen 15 und 16, wie in den Figuren 1 und 3c dargestellt ist, ein Gehäuse 7 aus. Dieses ist in der gezeigten Ausführungsform gegenüber der Umgebung im Wesentlichen geschlossen. In dem Gehäuse 7 ist die Feder 4 aufgenommen.

Sowohl der erste Gehäuseabschnitt 5 als auch der zweite Gehäuseabschnitt 6 sind in Form einseitig geschlossener Hohlzylinder jeweils mit einer zylinderförmigen Wand 8a, b und einer im Wesentlichen geschlossenen Stirnseite 9a, b ausgebildet. Im Zentrum der einen Stirnseite 9a des ersten Gehäuseabschnitts 5 des ersten Maulteils 15 ist ein Achsstift 10 angeordnet oder ausgebildet, der nach innen in das Gehäuse 7 hineinragt. Im Zentrum der Stirnseite 9b des Gehäuseabschnitts 6 des zweiten Maulteils 16 ist dem Achsstift 10 gegenüberliegend und zu diesem passend eine Öffnung (Loch) 17 ausgebildet. Nach erfolgtem Zusammenfügen des Maulteils 15 mit dem Maulteil 16 (siehe in den Figuren 1 und 3c) greift der Achsstift 10 in die Öffnung 17 ein und ist in dieser um seine Längsachse drehbar geführt. Die Längsachse des Achsstifts 10 bildet in der dargestellten Ausführungsform eine Drehachse 3 des Clips, um die die beiden Maulteile 15 und 16 relativ zueinander schwenkbar sind.

Die Innenseite der zylinderförmigen Wand 8a des ersten Gehäuseabschnitts 5 ist an ihrer zum Achsstift 10 weisenden Seite in umlaufender Richtung glatt ausgebildet. An der zum Achsstift 10 weisenden Innenseite der zylinderförmigen Wand 8b des zweiten Gehäuseabschnitts 6 ist ein Vorsprung 19 ausgebildet. Dieser ragt von der Innenseite der Wand 8b in das Innere des Gehäuseabschnitts 6 und damit des Gehäuses 7 hinein und erstreckt sich in orthogonaler Richtung bezogen auf die Stirnseite 9b des Gehäuseabschnitts 6 über die Höhe der Wand 8b hinaus. Die Höhe des Vorsprungs 19 ist derart bemessen, dass sein über die Wand 8b des zweiten Gehäuseabschnitts 6 ragender Teil gleich oder leicht kleiner als die Höhe der Wand 8a des ersten Gehäuseabschnitts 5 gemessen an deren Innenseite ist, so dass sich der Vorsprung 19 bei zusammengefügten Maulteilen 15,16 im Wesentlichen über die gesamte Innenhöhe des Gehäuses 7 erstreckt.

Der Achsstift 10 weist einen Schlitz 11 auf. Dieser ist in der Längsrichtung des Achsstifts 10, d.h. in Richtung der Drehachse 3 des Clips, durchgehend ausgebildet und dient als Aufnahme für die Feder 4. Ein Endabschnitt 14 der Feder 4 ist in den Schlitz 11 eingeschoben und darin zumindest verdrehsicher gehalten, so dass der Endabschnitt 14 drehfest mit dem ersten Maulteil 15 verbunden ist. Die Länge des Achsstifts 10 ist im Hinblick auf die beiden Gehäusehälften oder -abschnitte 15 und 16 derart bemessen, dass sein freies Ende bei vollständig zusammengefügten Maulteilen 15,16 in die Öffnung 17 hineinragt. Die Durchmesser von Achsstift 10 und Öffnung 17 sind derart ausgebildet, dass der Achsstift 10 darin verdrehbar geführt ist. Der Vorsprung 19 am Gehäuseabschnitt 6 bildet einen Anschlag für das dem Endabschnitt 14 gegenüberliegende freie Ende 20 der Feder 4 aus.

Auf der dem Klemmarm 1 radial gegenüberliegenden Seite des Gehäuseabschnitts 5 ist ein Betätigungsvorsprung 12 quasi in (gewinkelter) Verlängerung zum Klemmarm 1 ausgebildet, der von der Außenseite der Wand 8a radial nach Außen vorsteht. Auf der dem Klemmarm 2 radial gegenüberliegenden Seite des Gehäuseabschnitts 6 ist in entsprechender Weise ein Betätigungsvorsprung 13 ausgebildet, der von der Außenseite der Wand 8b radial nach außen vorsteht. Die Betätigungsvorsprünge 12, 13 bilden Ansätze oder Tasten aus, mittels denen der Clip gegen die Spannung der Feder 4 manuell geöffnet werden kann. Sie begrenzen außerdem aufgrund ihrer jeweils gewinkelten Ausrichtung zum zugehörigen Klemmarm die maximale Öffnungsweite des Clips, so dass ein Überdrehen der Feder 4 vermieden wird und eine ausreichend lange Standzeit der Feder 4 gesichert ist.

In der dargestellten Ausführungsform überkreuzen sich die beiden Maulteile 15, 16 des Clips scherenartig, so dass ein Öffnen des Clip erfolgt, indem die Vorsprünge 12 und 13 voneinander fort bewegt oder auseinander gedrückt werden. In einer anderen Ausführungsform, die in den Figuren nicht dargestellt ist, überkreuzen die Maulteile 15, 16 einander nicht, sondern verlaufen nebeneinander. In diesem Fall erfolgt ein Öffnen des Clips, indem die Vorsprünge 12 und 13 aufeinander zu bewegt oder zusammengedrückt werden. Die beiden vorgenannten Ausführungsformen unterscheiden sich daher durch die Wicklungsrichtung der Feder 4.

Die Feder 4 ist in den in den Figuren dargestellten Ausführungsformen in Form einer archimedischen Spirale mit einem konstanten Spiel zwischen aktiven Windungen ausgebildet. Sie besteht aus einem flachen Band rechteckigen Querschnitts. Die Breite der Feder 4 ist gleich oder leicht kleiner als die Innenhöhe des Gehäuses 7, so dass sie beidseitig an den Stirnseiten 9a, b geführt und abgestützt ist. Das freie Ende 20 der Feder 4 liegt vollflächig an dem Vorsprung 19 stirnseitig an, so dass eine optimale Abstützung der Feder 4 auf möglichst großer Fläche erreicht wird. Die Endfläche des freien Endes 20 der Feder 4 ist orthogonal bezogen auf deren Wicklungsrichtung ausgebildet. Entsprechend ist der Bereich des Vorsprungs 19 ausgebildet, an dem das freie Ende 20 abgestützt ist. Auch die Führung der Feder 4 über den Endabschnitt 14 erfolgt orthogonal zu Wicklungsrichtung, so dass bei einem Spannen der Feder 4 bei einem Öffnen des Clips ein Einleiten von Querkraftkomponenten in die Feder 4 vermieden und diese rein in Richtung der Wicklungen (in Aufweitrichtung) belastet wird. Die Feder 4 ist im Clip symmetrisch ausgebildet und angeordnet, insbesondere symmetrisch zu der in Längsrichtung der Klemmarme 1 und 2 verlaufenden Mittelachse der Klemmarme 1 und 2 ausgebildet und angeordnet. Auf diese Weise sind eine symmetrische Belastung und Verformung der Feder 4 quer zur Drehachse 3 des Clips bzw. der scharnierartig gekoppelten Maulteile 15, 16 sowie eine symmetrische Belastung der Maulteile 15 und 16 durch die Federkraft sichergestellt. Ein Verkanten der Maulteile 15 und 16 und damit der Klemmarme 1 und 2 mit deren Klemmflächen 18a, b ist nicht möglich und ein sicheres und flächiges Aufbringen der beabsichtigten Klemmkräfte sowie ein paralleles Öffnen der Klemmarme 1 und 2 gewährleistet.

Aufgrund der vorbeschriebenen Ausbildung des Clips ist dessen Montage einfach und ohne Verwendung zusätzlicher Werkzeuge möglich. Die Figuren 3a, 3b und 3c zeigen schematisch unterschiedliche Stadien des Zusammenfügens der beiden Maulteile 15 und 16. Die Feder 4 ist in dem in Fig. 3a gezeigten Stadium in den ersten Gehäuseabschnitt 5 des ersten Maulteils 15 eingelegt. Dabei ist deren Endabschnitt 14 in dem Schlitz 11 des Achsstifts 10 aufgenommen, so dass die Feder 4 gegenüber dem ersten Maulteil 15 gegen Verdrehen gesichert ist.

Das zweite Maulteil 16 wird auf das erste Maulteil 15 mit der in der zugehörigen Gehäusehälfte angeordneten Feder 4 lose aufgelegt. Dabei sind die Maulteile 15 und 16 in Richtung der Drehachse 3 voneinander distanziert. Die Klemmarme 1 und 2 sind um die zukünftige Schwenkachse verdreht versetzt und können aus der in Fig. 3a dargestellten Stellung, in der die Maulteile einander nicht kreuzen, sondern nebeneinander verlaufen, in die in den Fign. 3b und 3c dargestellten Stellungen gebracht werden, in der die Maulteile einander scherenartig queren. Bei dieser Drehbewegung der Maulteile 15 und 16 in die einander überkreuzende Anordnung werden die Klemmarme 1 und 2 um die Drehachse 3 verschwenkt und aneinander vorbei bewegt. Während der Abstand der Maulteile 15 und 16 in Richtung der zukünftigen Drehachse 3 des Clips die vorstehend beschriebene Bewegung der Klemmarme 1 und 2 aneinander vorbei ermöglicht, steht das freie Ende 20 der Feder 4 bereits mit dem Vorsprung 19 in Teileingriff. Bei dem vorstehend beschriebenen Verdrehen der beiden Maulteile 15 und 16 relativ zueinander erfolgt daher gleichzeitig ein Vorspannen der Feder 4.

Die Stärke der Feder 4 sowie die Ausrichtung des Schlitzes 11 des Achsstifts 10 und des Vorsprungs 19 zueinander bestimmen die Vorspannung der Feder 4. Die gewünschte Vorspannung der Feder 4 ist erreicht, sobald die Maulteile 15 und 16 aus der in Fig. 3a gezeigten Position in die in Fig. 3b gezeigte Position bewegt sind, in der die Klemmarme 1 und 2 gerade vollständig übereinander bewegt wurden und einander nicht mehr überdecken. Aus dieser Position erfolgt der Abschluss des Zusammenbaus der Maulteile 15 und 16, indem diese in Richtung der zukünftigen Drehachse 3, hier in Richtung des Achsstifts 10, zusammengeschoben werden, bis die Klemmflächen 18a, b der beiden Klemmarme 1 und 2 vollflächig über-/aufeinander liegen. In dieser Position sind die Maulteile 15 und 16 auch in axialer Richtung zueinander geführt. Die Stirnseiten 21 a, b der zylinderförmigen Wände 8a,b liegen in dieser Position, also nach abgeschlossenen Zusammenfügen der Maulteile 15 und 16, aneinander an, so dass das die Feder 4 umgebende Gehäuse 7 (im Wesentlichen) geschlossen ist. Die Feder 4 ist komplett durch das Gehäuse 7 geschützt und eine versehentliche Beschädigung nicht möglich. Der Clip weist eine im Wesentlichen geschlossene, glatte äußere Oberfläche auf, was das Applizieren des Clips sowie dessen Reinigung vereinfacht.

In den gezeigten Ausführungsformen können die Feder 4 einerseits und die Maulteile 15 und 16 andererseits aus unterschiedlichen Werkstoffen bestehen. Die Maulteile 15 und 16 bestehen zum Beispiel aus Kunststoff oder Keramik und sind mittels Spritzguss hergestellt, während die Feder 4 aus einem üblichen Federstahl besteht.

## Patentansprüche

1. Chirurgischer Clip mit einem ersten Klemmarm (1) und einem zweiten Klemmarm (2), die drehbar miteinander gekoppelt sind und mittels einer Feder (4) in einer Drehrichtung gegeneinander in eine geschlossene Stellung vorgespannt sind, wobei an dem ersten Klemmarm (1) ein erstes Gehäuseteil (5) und an dem zweiten Klemmarm (2) ein zweites Gehäuseteil (6) ausgebildet oder angeordnet sind, welche in Kooperation ein Gehäuse (7) ausbilden, in dem die Feder (4) aufgenommen ist, **dadurch gekennzeichnet, dass** die Feder (4) eine Spiralfeder in Form einer ebenen Spirale ist.

2. Chirurgischer Clip nach Anspruch 1, **dadurch gekennzeichnet, dass** das erste Gehäuseteil (5) an dem ersten Klemmarm (1) proximal endseitig ausgebildet oder angeordnet ist und/oder das zweite Gehäuseteil (6) an dem zweiten Klemmarm (2) proximal endseitig ausgebildet oder angeordnet ist.

3. Chirurgischer Clip nach Anspruch 1 oder 2, **dadurch gekennzeichnet, dass** das erste Gehäuseteil (5) und/oder das zweite Gehäuseteil (6) aus einer zylinderförmigen Wand (8a,b) mit jeweils einer geschlossenen Stirnseite (9a,b) ausgebildet sind.

4. Chirurgischer Clip nach einem der vorstehenden Ansprüche, **dadurch gekennzeichnet, dass** dieser einen Achsstift (10) aufweist, der im Inneren des Gehäuses (7) vorzugsweise zentral ausgebildet oder angeordnet ist.

5. Chirurgischer Clip nach Anspruch 4, **dadurch gekennzeichnet, dass** der Achsstift (10) die Drehachse (3) eines Scharniers ausbildet, über das der erste Klemmarm (1) und der zweite Klemmarm (2) miteinander drehbar gekoppelt sind.

6. Chirurgischer Clip nach Anspruch 4 oder 5, **dadurch gekennzeichnet, dass** der Achsstift (10) eine Aufnahme, vorzugsweise in Form einer Ausnehmung oder eines Schlitzes (11), aufweist, in der ein Endabschnitt (14) der Feder (4) gehalten ist.

7. Chirurgischer Clip nach einem der Ansprüche 4 bis 6, **dadurch gekennzeichnet, dass** der Achsstift (10) zu einem der beiden Gehäuseabschnitte (5,6) drehfest und zum anderen der beiden Gehäuseabschnitte (5,6) bezogen auf die Drehachse (3) drehbar ist.

8. Chirurgischer Clip nach Anspruch 3, **dadurch gekennzeichnet, dass** die zylinderförmige Wand (8a, b) des ersten Gehäuseteils (5) und des zweiten Gehäuseteils (6) jeweils Lagerflächen oder -abschnitte aufweisen, die ineinander greifen oder zusammenwirken, um ein Scharnier auszubilden, über das der erste Klemmarm (1) und der zweite Klemmarm (2) miteinander drehbar gekoppelt sind.

9. Chirurgischer Clip nach einem der vorhergehenden Ansprüche, **gekennzeichnet durch** Betätigungselemente (12,13) zum Öffnen des Clip gegen die Vorspannung der Feder (4), die vorzugsweise an dem ersten Gehäuseteil (5) und/oder dem zweiten Gehäuseteil (6) angeordnet oder ausgebildet sind.

10. Chirurgischer Clip nach Anspruch 9, **dadurch gekennzeichnet, dass** die Betätigungselemente (12,13) einen maximalen Öffnungswinkel des Clip definieren.

11. Chirurgischer Clip nach einem der vorhergehenden Ansprüche, **dadurch gekennzeichnet, dass** die Feder (4) in Form einer Archimedischen Spirale ausgebildet ist.

12. Chirurgischer Clip nach einem der vorstehenden Ansprüche **dadurch gekennzeichnet, dass** die Feder (4) einen rechteckigen Querschnitt aufweist.

## Claims

1. Surgical clip with a first clamping arm (1) and a second clamping arm (2), rotatably coupled with each other and pre-tensioned against each other into a closed position in a rotation direction by means of a spring (4), whereby a first housing part (5) is formed or arranged on the first clamping arm (1) and a second housing part (6) is formed or arranged on the second clamping arm (2), which cooperate to form a housing (7) in which the spring (4) is held, **characterised in that** the spring (4) is a spiral spring in the form of a level spring.

2. Surgical clip according to claim 1, **characterised in that** the first housing part (5) is formed or arranged on the first clamping arm (1) at the proximal end side and/or the second housing part (6) is formed or arranged at the proximal end side on the second clamping arm (2).

3. Surgical clip according to claim 1 or 2, **characterised in that** the first housing part (5) and/or the second housing part (6) is/are formed by a cylinder-shaped wall (8a, b) with a closed facing side (9a, b) each.

4. Surgical clip according to one of the preceding claims, **characterised in that** the same comprises an axial pin (10) that is preferably centrally formed or arranged in the interior of the housing (7).

5. Surgical clip according to claim 4, **characterised in that** the axial pin (10) forms the rotation axis (3) of a hinge, via which the first clamping arm (1) and the second clamping arm (2) are rotatably coupled with each other.

6. Surgical clip according to claim 4 or 5, **characterised in that** the axial pin (10) comprises a take-up, preferably in the form of a recess or a slot (11), in which an end section (14) of the spring (4) is held.

7. Surgical clip according to one of the claims 4 to 6, **characterised in that** the axial pin (10) is non-rotatable to one of the two housing parts (5, 6) and rotatable to the other of the two housing parts (5, 6) in relation to the rotation axis (3).

8. Surgical clip according to claim 3, **characterised in that** the cylinder-shaped wall (8a, b) of the first housing part (5) and the second housing part (6) each comprise storage surfaces or sections that engage or cooperate with each other to form a hinge, via which the first clamping arm (1) and the second clamping arm (2) are rotatably coupled with each other.

9. Surgical clip according to one of the preceding claims, **characterised by** activating elements (12, 13) for opening the clip against the pre-tension of the spring (4), which are preferably arranged or formed on the first housing part (5) and/or the second housing part (6).

10. Surgical clip according to claim 9, **characterised in that** the activating elements (12, 13) define a maximum opening angle of the clip.

11. Surgical clip according to one of the preceding claims, **characterised in that** the spring (4) is designed in the form of an Archimedian spiral.

12. Surgical clip according to one of the preceding claims, **characterised in that** the spring (4) has a rectangular cross-section.

## Revendications

1. Clip chirurgical comprenant un premier bras de serrage (1) et un deuxième bras de serrage (2), qui sont couplés l'un à l'autre de manière à pouvoir tourner et qui sont précontraints dans une position fermée l'un par rapport à l'autre dans une direction de rotation au moyen d'un ressort (4),
sachant qu'une première partie de boîtier (5) est réalisée ou disposée au niveau du premier bras de serrage (1) et qu'une deuxième partie de boîtier (6) est réalisée ou disposée au niveau du deuxième bras de serrage (2), lesquelles réalisent en coopération un boîtier (7), dans lequel le ressort (4) est logé,
**caractérisé en ce que** le ressort (4) est un ressort hélicoïdal se présentant sous la forme d'une spirale plane.

2. Clip chirurgical selon la revendication 1, **caractérisé en ce que** la première partie de boîtier (5) est réalisée ou disposée côté extrémité de manière proximale au niveau du premier bras de serrage (1), et/ou **en ce que** la deuxième partie de boîtier (6) est réalisée ou disposée côté extrémité de manière proximale au niveau du deuxième bras de serrage (2).

3. Clip chirurgical selon la revendication 1 ou 2, **caractérisé en ce que** la première partie de boîtier (5) et/ou la deuxième partie de boîtier (6) sont réalisées à partir d'une paroi (8a, b) de forme cylindrique avec respectivement un côté frontal (9a, b) fermé.

4. Clip chirurgical selon l'une quelconque des revendications précédentes, **caractérisé en ce que** celui-ci présente une tige axiale (10), qui est réalisée ou disposée de préférence de manière centrale à l'intérieur du boîtier (7).

5. Clip chirurgical selon la revendication 4, **caractérisé en ce que** la tige axiale (10) réalise l'axe de rotation (3) d'une charnière, par l'intermédiaire de laquelle le premier bras de serrage (1) et le deuxième bras de serrage (2) sont couplés l'un à l'autre de manière à pouvoir tourner.

6. Clip chirurgical selon la revendication 4 ou 5, **caractérisé en ce que** la tige axiale (10) présente un logement, de préférence sous la forme d'un évidement ou d'une fente (11), dans lequel une section d'extrémité (14) du ressort (4) est maintenue.

7. Clip chirurgical selon l'une quelconque des revendications 4 à 6, **caractérisé en ce que** la tige axiale (10) est solidaire en rotation par rapport à l'une des deux parties de boîtier (5, 6) et peut tourner par rapport à l'axe de rotation (3) par rapport à l'autre partie de boîtier (5, 6).

8. Clip chirurgical selon la revendication 3, **caractérisé en ce que** la paroi (8a, b) cylindrique de la première partie de boîtier (5) et de la deuxième partie de boîtier (6) présentent respectivement des surfaces ou des sections de palier, qui s'imbriquent les unes dans les autres ou coopèrent afin de réaliser une charnière, par l'intermédiaire de laquelle le premier bras de serrage (1) et le deuxième bras de serrage (2) sont couplés l'un à l'autre de manière à pouvoir tourner.

9. Clip chirurgical selon l'une quelconque des revendications précédentes, **caractérisé par** des éléments d'actionnement (12, 13) servant à ouvrir le clip à l'encontre de la précontrainte du ressort (4), qui sont disposés ou réalisés de préférence au niveau de la première partie de boîtier (5) et/ou au niveau de la deuxième partie de boîtier (6).

10. Clip chirurgical selon la revendication 9, **caractérisé en ce que** les éléments d'actionnement (12, 13) définissent un angle d'ouverture maximal du clip.

11. Clip chirurgical selon l'une quelconque des revendications précédentes, **caractérisé en ce que** le ressort (4) est réalisé sous la forme d'une spirale d'Archimède.

12. Clip chirurgical selon l'une quelconque des revendications précédentes, **caractérisé en ce que** le ressort (4) présente une section transversale rectangulaire.
